Europäisches Patentamt

European Patent Office

Office européen des brevets

(19)

(11) Publication number: **0 093 262**

**A1**

(12)

# EUROPEAN PATENT APPLICATION

(21) Application number: **83103019.2**

(22) Date of filing: **16.07.79**

(51) Int. Cl.³: **A 61 L 9/12**
**A 01 N 25/18, A 01 M 1/20**

(30) Priority: **10.05.79 US 37688**

(43) Date of publication of application:
**09.11.83 Bulletin 83/45**

(84) Designated Contracting States:
**BE CH DE FR GB IT NL**

(60) Publication number of the earlier application
in accordance with Art. 76 EPC: **0 019 010**

(71) Applicant: **Hyman, Sy**
**425 East 38th Street**
**New York, N.Y. 10022(US)**

(72) Inventor: **Hyman, Sy**
**425 East 38th Street**
**New York, N.Y. 10022(US)**

(74) Representative: **Schubert, Siegmar, Dipl.-Ing.**
**Patentanwälte Dr. V. Schmied-Kowarzik Dr. P. Weinhold**
**Dr.-Ing. G. Dannenberg Dr. D. Gudel Dipl.-Ing. S.**
**Schubert Dr. P. Barz Grosse Eschenheimer Strasse 39**
**D-6000 Frankfurt am Main 1(DE)**

(54) Article for the dispersion of a volatile substance and an article of manufacture comprising a plurality of said articles.

(57) In an article for the dispersion of a volatile substance to the surrounding atmosphere opposed outer and inner wall members (24, 32) are joined along their peripheral portions (12, 14, 20, 22) to define a centrally disposed reservoir portion (40). This reservoir portion contains the volatile substance (42). The outer wall comprises a liquid impervious gas permeable polymer (28). The first barrier layer (26) is removable attached to and covers the outer surface of this gas permeable polymer. The inner wall (32) comprises a second barrier layer (34) impervious to gases and liquids. The bond between the outer wall or gas permeable polymer (28) and the first barrier layer (26) is adapted to be weakened by the volatilizable substance dispersing through the outer wall. Thereby the ready removal of the first barrier layer from the outer wall is reached prior to use of the article.

Fig.3

Fig. 4

0093262

March 25, 1983          HYMAN, SY

SS/Ha                   Divisional of EP 79 102 485.4

Article for the dispersion of a volatile substance and an article of manufacture comprising a plurality of said articles

The invention relates generally to an article of manufacture for the slow release of a volatilized substance such as deodorizing means, insecticides, repellants, perfumes, medicaments and the like.

A prior art article for the dispersion of a volatile substance to the surrounding atmosphere has two laminated panels each comprising a plurality of layers (US-A-4 145 001). A first panel encompasses a substantially liquid impervious, gas permeable polymeric inner layer. Adjacent this layer of thermoplastic material are arranged a release layer, a vapor impermeable outer layer and a protective layer. The release layer and the vapor impermeable outer layer are bonded by a laminant. A second laminant is provided between the outer layer and the protective layer. The protective layer can be formed of Mylar polyester. The second panel which is designed impervious to molecular diffusion comprises a second inner layer which may be formed of the same material as the gas permeable layer but it is not necessary that the second inner layer be permeable to the vapors of the volatile material. The second panel further encompasses an outer layer of aluminium, a protective layer and a laminant between the outer layer and the protective layer. - A volatilizable substance is contained in an absorbant pad which is arranged between both panels. The permeable inner layer is initially bonded to the release layer which consists of a material not forming strong heat bonds with the permeable layer. However the strength of the heat bonds remains essentially constant independent of the time after finishing the heat bonds. Therefore a compromise has to be chosen for providing a heat bond strong enough which is not impaired by the manufacturing process and by the handling immediately following the manufacturing process but which is nevertheless weak enough for easy release prior to use of the article for the dispersion of the volatile substance. Further, the panels constituting this complex layer system are expensive to manufacture.

It is therefore an object of the invention to improve the connection between the barrier layer (or release layer) which has to be released from the gas permeable layer prior to activating the article. The release shall be effected easily although a reliable connection shall be achieved for lasting the time in which the article suffers the strongest impact in the course of manufacturing and immediately following thereto. It is another object of the invention to provide an article with an uncomplicated design of the layers. More generally it is an object of the invention to provide such an article embodying structial features enabling efficient utilization of volatile substance and only at time of desired use.

Yet a still further object of the invention is to provide such an article having an arrangement of parts conductive to efficient mas production and relatively inexpensive manufacture.

According to the invention the bond between the outer wall established by the gas permeable polymers and the first barrier layer is adapted to be weakened by the volatilizable substance dispersing through the outer wall. Thereby ready removal of the first barrier layer from the outer wall permits dispersion of the volatile substance through the outer wall to the surrounding atmosphere.

However, during manufacturing and transport steps in the course of the manufacturing and immediately following manufacturing the bond between the outer wall and the barrier layer is relatively strong and will not be impaired by the handling of the article.

It is particularly advantageous to dispose an adhesive which is attackable by the volatilizable substance between the gas permeable polymeric layer and the first barrier layer. - Due to the properties of this adhesive the adhesive bond between the polymeric layer and the barrier layer will be attacked and degraded in the course of the time so that this bond is relatively weak not prior to actual use of the article.

The article in which opposed outer and inner wall members are heat-sealed along their peripheral positions, the outer wall comprising a molecular diffusion layer and the inner wall comprising a barrier layer may be easily manufactured at low cost in that the second barrier layer comprises a dissimilar polymer from the polymer of the molecular diffusion layer. - Thereby additional layers between the barrier layer and the molecular diffusion layer can be avoided although a strong heat seal is effected along their peripheral positions.

The invention is described but not limited by reference to the accompanying drawing wherein:

FIG.  1 is a top plan view shown partly broken away illustrating one embodiment of the invention;

FIG.  2 is a side elevational view of the embodiment of FIG. 1;

FIG.  3 is an end sectional view taken along line 3-3 in FIG. 2;

FIG.  4 is a process diagram depicting the step-wise method of forming the article of the invention;

FIG.  5 is a plan view with partly broken away section illustrating another embodiment of the invention;

FIG.  6 is a side elevational view of the folded side;

FIG.  7 is an enlarged sectional view taken along line 7-7 of FIG. 6; and FIG. 7A is an alternate embodiment;

FIG.  8 (a)-(e) is a schematic illustration of the process for making the latter embodiment of FIGS. 5 - 7 and 10;

FIG.  9 is a side view taken along line 9-9 of FIG. 8;

FIG. 10 is a sectional perspective view of the embodiment of FIGS. 5 - 7 and

FIG. 11 and 11A show another embodiment of the invention.

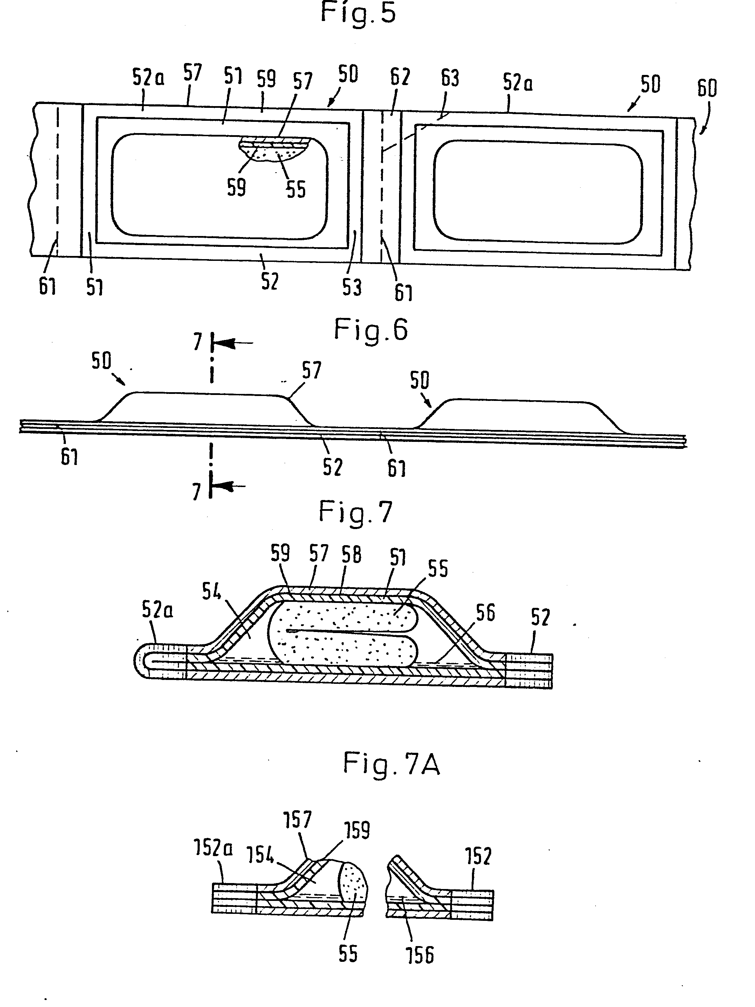

In the latter embodiment shown in FIGS. 5 - 10, it is a further object of the present invention to provide a slow vapor release article wherein single gas permeable polymeric material is employed in forming the reservoir and yet the aforesaid embodiments are achieved.

It is still a further object of this invention to provide an article as immediately aforesaid in which a solid pad provides the volatilizable liquid source, solid block, pellets or gels.

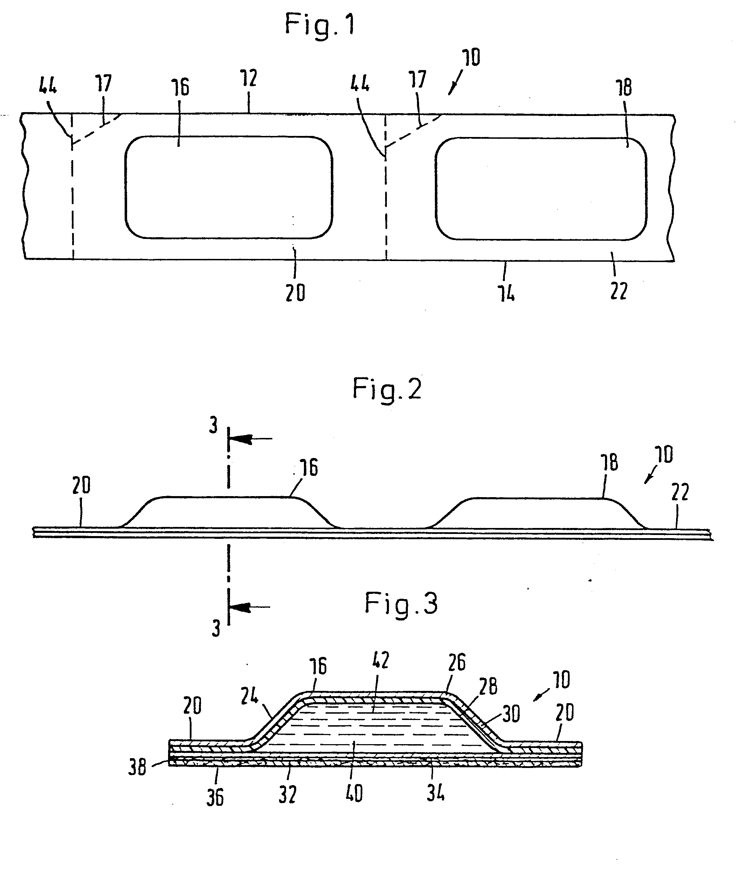

In the following description like reference numerals designate similar parts throughout the several views and wherein 10 generally designates an article in accordance with the invention, the embodiment of FIG. 1 depicting an elongated strip material having opposed longitudinal edges 12 and 14 provided with pouch or envelope portions 16 and 18 surrounded by sealed rim portions 20 and 22, respectively. The construction is illustrated in more detail in FIG. 3. Outer wall member 24 comprises a first barrier layer 26 which completely overlies polymeric diffusion layer 28 being removably adhered thereto. Vapor barrier layer 26 may be of a variety of materials but is most preferably thin metal foil such as aluminium foil. It is essentail in the present invention that vapor barrier layer 26 be impermeable to the passage of gas or vapor as will be described in detail hereinafter.

Materials other than metal foil such as a wide variety of plastic materials, may be effectively used provided such material have the defined vapor impermeability characteristics and particularly within the temperature-humidity ranges encountered in the intended use. For example, as regards deodorizing applications in accordance with the invention, moderate temperatures normally characteristic of household applications would be encountered; however, in accordance with other embodiments of the invention e.g., the use of the article as a deodorizer, sanitizer, etc. to be added to the drying cycle of a clothes laundering operation, the temperatures would be quite higher. Thus, the chemical nature of barrier layer 26 depends on the ultimate utility contemplated, and selection of this material has necessary reference thereto.

Polymeric materials useful in the preparing diffusion layer 28 may be selected from a relatively wide range of substances, e.g., polymers and interpolymers derived the polymerization of one or more ethylenically unsaturated monomeric materials, i.e., the vinyl monomers. The polymeric materials are essentially water insoluble i.e., hydrophobic. In addition, such materials, in accordance with an essential aspect of the invention, while being of a impervious nature will nevertheless allow diffusion of the gas or vapor produced form the volatilization of the volatiled material confined within the reservoir 16. A particularly preferred polymer of the vinyl type is polyvinyl chloride although polyethylene, polypropylene and the like, may be used. Polymers other than those of the vinyl type may be used with advantage, these including, without necessary limitation, polyurethanes, polysiloxanes (silicones), thermoplastic elastomers, rubbers, etc. such as the product available commercially under the trade name designation HYTREL. Preferably the gas permeable polymeric layer is a polyethylene terephtalate marketed under the trademark MYLAR. As was the case with reference to barrier layer 26, selection of a given polymer within the limitations expressed is controlled at least in part by the conditions to be encountered in the ultimate use contemplated. The molecular weight of the polymer material may be considered in the selection of the diffusion layer. The thickness of polymer layer 28 will generally range between about .01 and .001 inch. In the case of metal foil, layer bonding thereof to polymer layer 28 may be achieved with adhesive; adhesive is also optional with the use of plastics as the barrier layer 26, the requiisite bonding being obtainable, for example, by heat seal or heat fusion.

It is to be borne in mind that while the metal foil prevents vapor release to the atmosphere, some volatile fraction permeates the polymer diffusion layer and collects between the foil-polymer interface thereby separating and permitting more ready release of the foil from the polymer up to the heat-sealed perimeter of the reservoir.

For manufacturing convenience inner wall member 32 may be same as outer wall member 24 previously described. Alternatively, the inner wall may comprise only a single layer 34 which may be constructed of the same or different material as barrier layer 26. Again the essential properties of such barrier layer is both liquid and gas permeable, in additional must be sealable to outer wall thereby completing formation of the sealed reservoir.

Barrier layer may be coated with an adhesive, preferably pressure sensitive, and an outer release paper. Alternatively, a section of double faced adhesive film and release paper may be applied, or tipped on during the reservoir forming, filling or sealing machine cycle.

Polymer diffusion layer 28 and barrier layer 34 are bonded to form a permanent union. This union is most effectively accomplished by heat-sealing or heat-fusion according to known techniques to form reservoir or supply portion 40 enclosed by rim portion 20. Reservoir portion 40 is adapted to contain and confine a volatile substance 42 which may be in liquid form, e.g. solution, gel emulsion, suspension, etc., or alternatively may comprise a solid shape pellets, or semi-solid material (not shown) such as wax, padding material and the like saturated or super-saturated with the volatile substance. Gel forms include blends of the liquid active volatile agents with gelling agents such as cab-o-sil, carinogen, carboxymethyl cellulose, and the like. Polymer layer 28 can be set to the partly arcuate shape illustrated in FIG. 3, e.g., by molding, or alternatively, can be a resiliently felxible member which assumes the shape illustrated upon introduction of the volatile substance.

Suitable volatile substances useful in the construction herein described include perufmes, deodorizers, fragrances, anti-bacterial agents, anti-microbial agents, counter-actants, insecticides, insect-repellants, corrosion-inhibitors, attractants, medicants and the like. Suitable perfums include without necessary limitation the essential oils of various fragrances, flower perfumes, e.g., lilacs, roses, etc., wood perfumes such as cedar, pine, briar, etc., fruit flavorings such as lemon, cherry, etc..

Medicinal volatiles include menthol, camphor, eucalyptus and the like; exemplary anti-bacterial agents include phenol, thymol, and the like.

In use, the consumer merely removes barrier layer 26 to "activate" the vapor dispensing article. For such purposes, a tab member 17 may be provided on layer 26. Removal of layer 26 allows the vapor from volatile substance 42 to escape into the surrounding atmosphere through permeable polymeric layer 28. The vapors are dispensed at a substantially linear rate and penetrate the entire extent of the reservoir surface area of layer 28. Premature or inadvertent dispensing of the vapors is effectively prevented by virtue of barrier layer 26. Thus, until the time of actual use, barrier layer 26 remains in place which time removal is effected by the consumer. If desired, layer 26 can be repositioned on the polymer layer and the vapor diffusions discontinued until a later time.

In a further aspect of the invention, release layer 36 can also be stripped from teh article as desired by the consumer to expose adhesive layer 38. This adhesive backing enables the article to be attached to a variety of environmental surfaces such as ables, closets, bathroom surfaces, etc.. Moreover, being of flexible, lightweight construction, it can readily be affixed to surfaces of arcuate configuration such a piping. This adaptability feature enables the article to be positioned in areas which would not oridnarily be readily visible and is thus of significant advantage from an esthetic standpoint. The use of perfume-containing articles is of particular value where it is desired to mask or neutralize objectionable scents such as those encountered in kitchens, bathrooms, closets and the like. The article may also be used to dispense vapors having therapeutic effects when inhaled and can accordingly be affixed to any suitable surface in the room where necessary.

Handling of the present article is expedited by the presence of rim portion 20 (FIG. 3). Thus in use, the consumer need not directly contact pouch portion 16 and thus indirectly, the contents of reservoir portion 40. This is important since it virtually eliminates inadvertent rupturing of the pouch portions which might otherwise occur due to the compression force exerted in handling. As will be further noted, escape of the volatilized material is limited directionally, outwardly of the element through gas-permeable polymeric layer 28. Barrier layer 34 prohibits escape of such vapors rearwardly of the element. It is important that barrier layer 26 completely cover the outer reservoir surface of polymeric layer 28 so as to provide a seal. In this manner, premature escape of vapor is prevented.

As illustrated in FIG. 1, the article of the present invention may be provided in the form of an elongated strip comprising a plurality of such articles. Perforations 44 provided laterally of the connecting rim portions enable individual elements to be severed as desired. Alternatively, several connected elements can be used to provide an even greater supply of vapor to the surrounding atmosphere.

As previously mentioned, the volatile material may be in the form of a liquid, e.g., solution, suspension or emulsion, or in the form of a semi-solid or solid material impregnated with the volatile material. Thus, the emulsion form can be achieved with the use of a suitable solvent along with a surfactant material and/or a protective colloid such as polyvinyl alcohol, methyl cellulose and the like. Particularly effective surfactants include nonionic detergents derived from the polyoxyalkylation of alcohols. Impregnated waxes and fibrous materials may also be used. In any event, regardless of the type of carrier used, the volatile substance should readily produce vapor for passage through polymeric diffusion layer 28.

One of the particularly valuable aspects of the invention relates to the fact that a relatively large volume, and high concentration of aromatic perfume, for example is accommodated by the article reservoir relative to its total size, e.g. 3 - 4 grams of perfume concentrate per article. In addition, an almost linear release of the perfume is achieved due to the constant concentration gradient of volatilizable material maintained within reservoir portion 40. This allows substantially uniform dispensing of the perfume ingredient over a prolonged period of use. This is to be contrasted with prior art devices and particularly those requiring adjustment of the part of the user as a given increment of available surface area sublimes and becomes depleted. Furthermore, the present articles are relatively inexpensive due to the simplicity of their construction, ease of continuous manufacture and the low cost types of polymeric material required.

In the present invention, the hermetically sealed envelope type construction renders the article highly efficient as regards the use of highly concentrated reservoir supply.

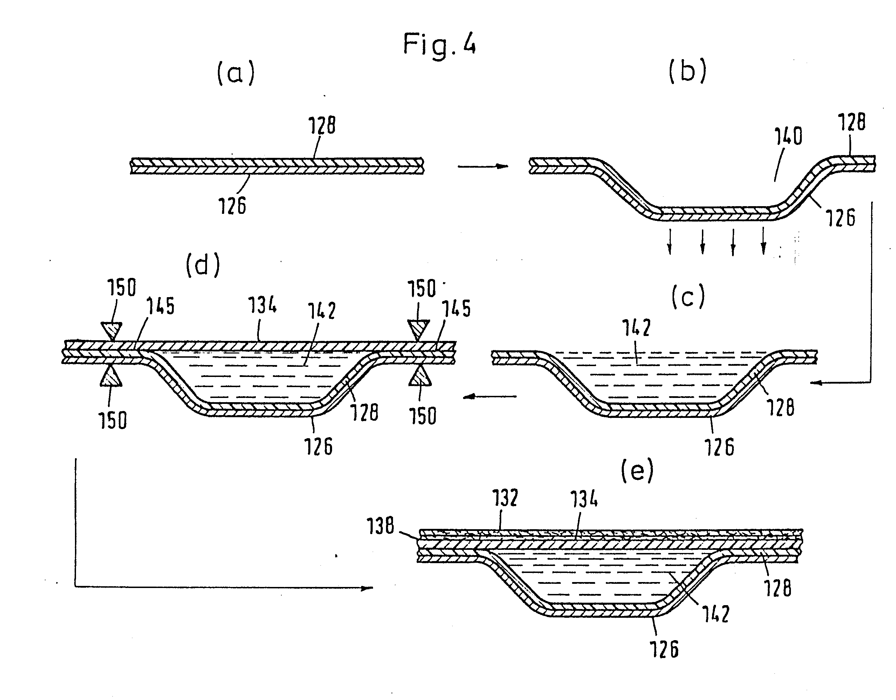

Referring now to FIG. 4. there is shown a step-wise method of forming the article of the present invention. In step a., a continuous strip of the diffusion polymer 128 bonded to the metal foil 126 is provided with the metal foil disposed below the polymer, and in step b., a pouch or reservoir 140 is formed in the strip by well-known pressure-forming or vacuum forming means (not shown). In step c., the volatile substance (e.g. liquid) 142 is placed in formed reservoir 140. Thereafter a strip of polymer barrier layer 134 is placed over the reservoir portion as well as over the face portions 145 of polymer diffusion layer 128, and a well-known heat-seal crimp mechanism 150 provides a peripheral heat-seal of polymer layers 134 and 128, and a crimp-seal of layer 128 to foil 126. Turning to step c., the article of step d., is further provided with a pressure sensitive adhesive covered by release paper 132.

The present articles may be simply manufactured. As stated, bonding of polymeric layer 128 and barrier layer 34 can be effected by simple heat sealing of fusion. Volatile liquid substance can thereafter be introduced into reservoir portion 40 by needle injection. In the case of solid and semi-solid volatiles, fusion of layers 28 and 34 may be effected across the mass of volatile material. Other techniques relevant to preparation of the present articles are described for example in U.S. patents 2 940 230; 2 793 481; 2 290 564; 3 020 687; 2 958 169; 2 998 176; 2 469 975; 2 802 324; 3 065 915 and 636 317.

Referring to FIGS: 5- 10 there is shown another embodiment of the invention generally referred to by numeral 50. Article 50 comprises a liquid impermeable gas permeable polymeric sheet 51 which is folded on itself as at 50 and heat sealed at four portions, 52a, 51, 52 and 53 so as to form a liquid reservoir 54. A saturated fabric or paper pad 55 is disposed within the reservoir 54. An aluminium foil 57 has previously been adhesively bonded to the outer surface 58 of the polymer as at 59 and extends around the polymer. The volatilizable material, diffuses through the polymer 51 and renders the foil barrier layer more readily peeled and removed from the polymer layer to activate the article.

The article 50 is preferably formed as a strip 60 which contains a plurality of said articles 50. The strip contains a plurality of scores of perforations 61 which permit detachment of the individual articles 50 from each other. Each article also contains a partial oblique score 63, to provide a tab 62 for readily lifting the foil 57 in peeling same.

It is to be understood that the foil need only be peeled to the extent one would want to expose the polymer surface.

The more surface area exposed the more volatile material disperses to the atmosphere for the same time period.

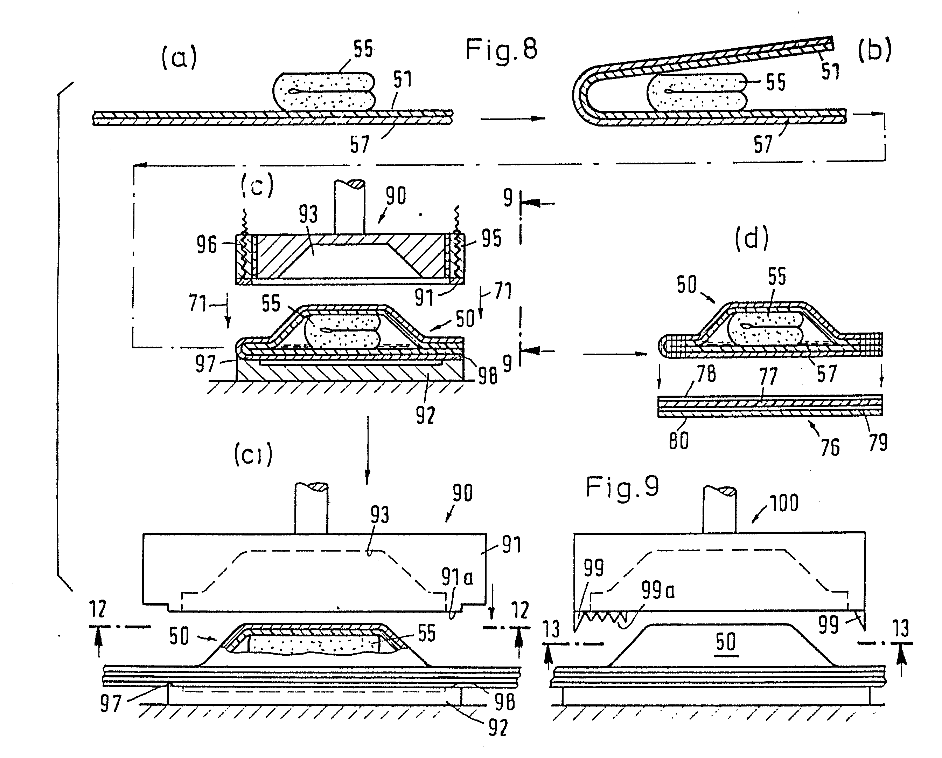

Referring to FIG. 8, there is shown in FIGS. 8(a) to 8(d) a process schematic for forming the embodiment of FIGS. 5 - 7; and FIG. 8(e) refers to a further embodiment. In FIG. 8(a) there is shown the strip 60 in cross-section (end view) wiht aluminium foil 57 adhesively bonded thereto. Pad 55 is disposed on the polymer about one-quarter of the distance transversely across the polymer surface.

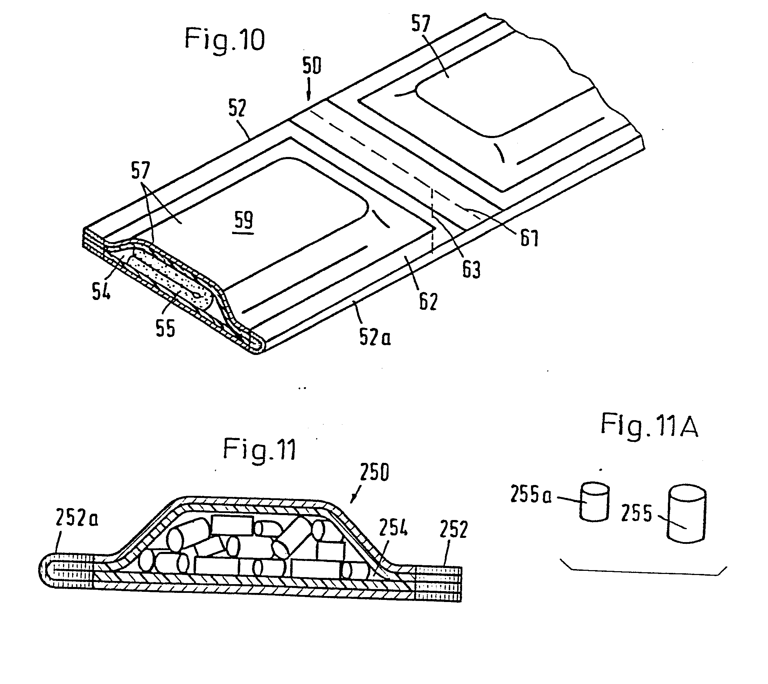

The polymer-foil sheet is then folded over to cover the pad as shown in FIG. 8(b). In-line heat-seal and perforating and scoring elements 90 and 100 respectively are well known in the art, and are applied as shown in FIGS. 8(c) - (c₁) and 8(d) respectively, so as to form sealed

perforated portions 51, 52, 53 and 54 and scored portions 61 and 63. It is to be noted that transverse score 61 passes through the entire profile while oblique or tab score 63 does not, as best shown in FIG. 10. The partial (to 62a) score 63 permits ready peel-away of the barrier layers. The scores 61 and 63 are formed by score elements 99 and 99a, respectively (FIGS. 9 and 13). A score-receiver platen 101 is provided to facilitate scoring.

In further operation A, the article 50 may be further processed as shown in FIG. 8(d). Specifically, there is shown a double adhesive tape 76 comprising strip 77 with adhesives 78 and 79 on opposing surfaces, a peelable paper strip 80 covering adhesive 79 which tape may be adhesively of thermoadhesively bonded to the under surface 57. In this manner of construction the paper 80 may be peeled and the article 50 then applied to any desired surface.

Specifically in forming the sealed article, a die 90 is provided which has vertically reciprocating top member 91 in relation to the feed strip, and a stationary bottom platen 92 or more specifically member 91 is formed with a recess 93 to accommodate the shape of the article. Member 91 is also formed with rectilinear peripheral electrical heat sealing elements (e.g. 95, 96) which are oppositely disposed to platen elements 97 and 98 so as to form the sealed portions 52a, 51, 52 and 53 in the article. As stated, in-line post-forming is made with perforation die cutter elements 99 and 99a of cutter 100 for forming perforations or scores 61 and the tab score 63 for tab 62; said scoring means being well-known in the art.

Referring to FIG. 7A, there is shown a alternate embodiment 150 wherein two separate polymeric and foil layers are heat sealed along four rectilinear lines, with seals 152a and 152 being shown. Thus, instead of a single polymer-foil sheet being folded over, two separate opposed polymer-foil strips (e.g. top polymer 159 and foil 157) are heat sealed to form reservoir 154 for pad 155 and its minimal liquid 156.

Referring to FIGS. 11 and 11A, there is shown another embodiment 250 which comprises separate polymeric and foil layers heat sealed along four rectangular lines with seals 252 and 252a being shown, as is similar to that of FIG. 7A. The heat sealed reservoir 254 is similar in construction to that of 154 of FIG. 7A embodiment. However, the reservoir in this other embodiment contains solid pellets 255 and 255a. Pellets 255 and 255a are formed of a polymeric matrix (e.g. ethylene, vinyl acetate, polyvinyl chloride, polypropylene and the like) which matrix contains a predetermined concentration of volatilizable active agent.

It is to be noted that while cylindrical pellets 255 and 255a are shown, any solid-shaped particulates may be employed, including rods, spheres, flakes, powders and the like. The volatilizable active agent may be mixed with a polymeric matrix material and formed by extrusion or casting and converted to the desired size and shape as is well-known in the art.

The rate of release of volatilizable active agent from the polymer matrix can be predetermined by selecting polymer matrix composition, varying the concentration of active agent contained therein and varying the size (surface area) of the particulates.

It is also to be noted, pellets 255 and 255a are differently sized. Each pellet may contain a different active agent being released into the sealed reservoir at a predetermined rate. The combined vapors will move through the polymeric diffusion layer into the surrounding environment.

Great latitudes for achieving a programmed release of one or more volatilizable agents can be achieved by premixing or blending particulates containing active agents, and inserting a predetermined amount of acitve matrix into the reservoir of the dispenser.

The present invention has been described with respect to certain preferred embodiments thereof. However, it will be understood that changes and modifications may be made without departing from the scope thereof.

Claims:

1. An article for the dispersion of a volatile substance to the surrounding atmosphere, said article comprising opposed outer (24) and inner (32) wall members joined along their peripheral portions (12, 14, 20, 22) to define a substantially centrally disposed reservoir portion (40) for containing said volatile substance (42), said outer wall comprising said substantially liquid impervious, gas permeable polymer (28), and said first barrier layer (26), removable attached to and covering the outer surface thereof, said barrier layer being substantially impervious to gases and liquids, and said inner wall (32) comprising a second barrier layer (34) substantially impervious to gases and liquids, characterized in that the bond between said outer wall (gas permeable polymer 28) and said first barrier layer (26) is adapted to be weakened by the volatilizable substance dispersing through said outer wall, whereby ready removal of said first barrier layer from said outer wall permits dispersion of said volatile substance through said outer wall to the surrounding atmosphere.

2. An article according to claim 1, characterized in that an adhesive is disposed between said gas permeable polymeric layer (28) and said first barrier layer (26) which adhesive is attackable by said volatilizable substance.

3. An article for the dispersion of a volatile substance to the surrounding atmosphere, said article comprising opposed outer (126, 128) and inner (134) wall members heat-sealed along their peripheral positions to define a substantially centrally disposed reservoir portion (140) for containing said volatilizable substance (142), said outer wall comprising said molecular diffusion layer (128) and said first barrier layer (126) removably attached to and covering the outer surface thereof, said first barrier layer being substantially impervious to molecular diffusion, and said inner wall (134) comprising a second barrier layer substantially impervious to molecular diffusion, characterized in that said second barrier layer (134) comprises a dissimilar polymer from the polymer of said molecular diffusion layer (128).

Fig.1

Fig.2

Fig.3

Fig. 4

Fíg.5

Fig.6

Fig.7

Fig.7A

Fig.8

(a)

55
51
57

(b)

55
51
57

(c)

90
93
96
95
91
71
71
55
50
97
98
9
9
92

(d)

50
55
78
77
57
80
76
79

(c₁)

90
93
91
91a
12
12
50
55
97
98
92

Fig.9

100
99
99a
99
13
13
50

Fig.10

Fig.11

Fig.11A

## European Patent Office

## EUROPEAN SEARCH REPORT

Application number

EP 83 10 3019

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | GB-A-1 516 845 (DRG) <br> * Page 1, lines 77-96; figure 1 * | 1,3 | A 61 L 9/12 <br> A 01 N 25/18 <br> A 01 M 1/20 |
| D,X | US-A-4 145 001 (R.J. WEYENBERG) <br> * Column 8, example 8; column 2, lines 1-45 * | 1 | |
| X | US-A-4 157 787 (B. SWARTZ) <br> * Figure 1; column 2, lines 38-65 * | 3 | |

TECHNICAL FIELDS
SEARCHED (Int. Cl. 3)

A 61 L 9/12

The present search report has been drawn up for all claims

| Place of search <br> THE HAGUE | Date of completion of the search <br> 26-07-1983 | Examiner <br> PELTRE CHR. |
|---|---|---|

EPO Form 1503. 03.82